Europäisches Patentamt

(19) European Patent Office

Office européen des brevets

(11) Veröffentlichungsnummer: **0 163 851**
**A2**

(12) **EUROPÄISCHE PATENTANMELDUNG**

(21) Anmeldenummer: 85104218.4

(22) Anmeldetag: 06.04.85

(51) Int. Cl.⁴: **C 07 D 493/04**
// (C07D493/04, 307:00, 307:00)

(30) Priorität: 08.06.84 DE 3421301

(71) Anmelder: **HÜLS AKTIENGESELLSCHAFT, - RSP
Patente / PB 15 - Postfach 13 20, D-4370 Marl 1 (DE)**

(43) Veröffentlichungstag der Anmeldung: **11.12.85
Patentblatt 85/50**

(84) Benannte Vertragsstaaten: **AT BE CH DE FR GB IT LI NL**

(72) Erfinder: **Scharf, Helmut, Dr., Kilianstrasse 75,
D-4235 Schermbeck (DE)**

(54) **Verfahren zur Herstellung von Pyromellitsäuredianhydrid.**

(57) Die Erfindung betrifft ein Verfahren zur Herstellung von Pyromellitsäuredianhydrid in einem geschlossenen Gaskreislauf durch katalytische Gasphasenoxidation von 1,2,4,5-Tetraalkylbenzolen mit einem Sauerstoff enthaltenden Gas, wobei

a) die Desublimation des Pyromellitsäuredianhydrids mit Hilfe eines kalten, Sauerstoff enthaltenden Gases durchgeführt wird,

b) die in dem Abgas der Desublimation enthaltenden Nebenprodukte katalytisch verbrannt werden und

c) das Abgas der katalytischen Verbrennung der Nebenprodukte anteilig dem Reaktor für die Pyromellitsäure-dianhydrid-Herstellung zugeführt wird.

Verfahren zur Herstellung von Pyromellitsäuredianhydrid

Die Herstellung von Pyromellitsäuredianhydrid (PMDA) erfolgt großtechnisch nach zwei verschiedenen Verfahren. Bei der Flüssigphasenoxidation werden 1,2,4,5-Tetraalkyl-benzol oder 2,4,5-Trialkyl-benzaldehyde mit Salpetersäure oder sauerstoffhaltigen Gasen unter Verwendung von halogenhaltigen Metall-Katalysatoren umgesetzt. Hierbei wird nicht das gewünschte Dianhydrid, sondern die freie Säure erhalten, die erst in einem anschließenden Verfahrensschritt zum Dianhydrid dehydratisiert werden muß. Diese zusätzliche Verfahrensstufe und erhebliche Korrosionsprobleme machen diese Verfahren teuer und wenig attraktiv.

Bei der Gasphasenoxidation werden die 1,2,4,5-Tetraalkylbenzole zusammen mit Luft in der Gasphase über einen heterogenen Katalysator geleitet und das Dianhydrid desublimiert bei Abkühlen des Reaktionsgases. In einer verbesserten Ausführungsform wird das Reaktionsgas gemäß DE-PS 23 62 659 nach einer Vorkühlung auf eine Temperatur, die oberhalb der Kondensationstemperatur des PMDA liegt, an gekühlten und thermostatisierten Flächen abgekühlt, wobei das PMDA desublimiert. Die Temperatur der Kühlflächen muß unterhalb der Kondensationstemperatur des PMDA, aber oberhalb der Kondensationstemperatur der Nebenprodukte, liegen.

Nachteilig bei diesem und ähnlichen Verfahren, die gekühlte Oberflächen zum Desublimieren des PMDA verwenden, ist, daß große Kühlflächen nötig sind.

Eine andere Möglichkeit, PMDA aus dem Reaktionsgas zu desublimieren, besteht in der zusätzlichen direkten Abkühlung des Reaktionsgases mit kalten Gasen. So wird z. B. gemäß FR-A 2 082 822 zu dem Reaktionsgas in zwei hintereinander geschalteten Rohren kalte Luft hinzugegeben. Außer einer Verbesserung der Reinheit von 95 % ohne Verwendung von Kühlluft auf 97 % bei Verwendung von Kühlluft (Verhältnis Reaktionsgas : Luft = 1 : 1), ist bei dem vorgeschlagenen Verfahren keine Verbesserung der Bedingungen zu erkennen, denn die Wandung der Desublimation dient auch noch zur Wärmeabfuhr, und die Fläche der Wandung ist, da sie nicht aktiv gekühlt wird, sondern nur über die Wärmeabstrahlung wirkt, für eine technische Anlage viel zu groß. So würde z. B. die Wandung bei einer Anlage mit einem Durchsatz von 1 t Durol/h ca. 125 000 m$^2$ betragen. Durch die Einspeisung von Kühlluft wird nach dem Verfahren der FR-A 2 082 822 die Abscheidung des PMDA an der Wandung zwar verbessert. Dies bringt aber den Nachteil der schnelleren Verstopfung der Apparatur mit sich. Da bei diesem Verfahren gegenüber den vorher beschriebenen Verfahren eine wesentlich größere, in der Regel die doppelte Menge an Abgas gereinigt werden muß, wird das Problem der Beseitigung der Nebenprodukte aus dem Abgas noch verschärft. Zu diesen Nachteilen kommt noch hinzu, daß für das Gesamtverfahren die doppelte Menge an Luft, ein Teil für die Reaktion, ein Teil für die Desublimation, gebraucht wird, was das Verfahren verteuert. Aus all diesen Gründen ist dieses vorgeschlagene Verfahren technisch nie realisiert worden.

Das Problem der fraktionierten Desublimation tritt nicht nur beim PMDA, sondern auch bei der Herstellung anderer Stoffe auf. So wird z. B. gemäß GB-PS 1 081 579 ein Verfahren und eine Apparatur zur fraktionierten Desublimation von Terephthalsäure beschrieben. Bei diesem Verfahren wird das Reaktionsgas durch die direkte Kühlung eines unter den Desublimationsbedingungen gasförmigen Stoffes erreicht, wobei die Temperatur des gasförmigen Stoffes unter der Kondensationstemperatur des abzuscheidenden Produktes und über der Kondensationstemperatur der Nebenprodukte liegen muß. Die Wandung des Desublimators wird auf eine Temperatur oberhalb der gewählten Kondensationstemperatur eingestellt.

Als bevorzugtes Kühlmittel wird Wasser in flüssiger Form eingespritzt. Dies ist bei der Desublimation von PMDA nicht möglich, da sich an den Wassertröpfchen bereits vor ihrer vollständigen Verdampfung neben dem PMDA auch Nebenprodukte absetzen würden. Hinzu kommt, daß bei den großen Mengen an Wasser bzw. Wasserdampf bei den Temperaturen der PMDA-Desublimation PMDA zur Säure hydratisiert würde. Würde das Wasser durch Kühlgase ersetzt, so würden, wenn die vorgeschlagenen Wassermengen auf Gas umgerechnet werden, sehr große Mengen an Kühlgasen benötigt, da das Kühlgas infolge der großen Verweilzeit von 40 s in der Kondensationszone zum Teil von den Wänden aufgewärmt und nicht zur Abkühlung des Reaktionsgases zur Verfügung stehen würde. Die große Verweilzeit der Gasmischung in der Kondensationszone würde zudem bei der Übertragung des Verfahrens auf die PMDA-Herstellung technisch nicht handhabbare Apparate-Dimensionen bedeuten, und die sehr großen Abgasmengen würden eine sinnvolle Reinigung des Abgases unmöglich machen. Daher ist dieses Verfahren nicht auf die PMDA-Herstellung anwendbar.

Aufgabe der vorliegenden Erfindung war es daher, ein Verfahren zur Herstellung von PMDA nach dem Gasphasenverfahren zu entwickeln, bei dem die drei Verfahrensschritte Oxidation des Einsatzproduktes zu PMDA, Desublimation und Abgas-Aufarbeitung so gestaltet und miteinander verbunden werden, daß die pro Mengeneinheit PMDA benötigte Gasmenge insgesamt nicht oder nur unwesentlich größer ist als die für die Oxidation des Eduktes erforderliche Gasmenge.

Diese Aufgabe wird erfindungsgemäß dadurch gelöst, daß die Desublimation des PMDA mit Hilfe eines kalten Gases durchgeführt wird, die in dem Abgas der Desublimation vorhandenen Nebenprodukte katalytisch verbrannt werden und ein Teil des Abgases aus der katalytischen Verbrennung

0163851
O.Z. 3990

der Nebenprodukte dem Hauptreaktor für die Erzeugung von PMDA wieder zugeführt wird, so daß ein geschlossener Gaskreislauf entsteht, während der andere Teil des Abgases aus der Verbrennung der Nebenprodukte in die Atmosphäre abgegeben wird und/oder zur Desublimationsstufe unter Bildung eines zweiten geschlossenen Gaskreislaufes zurückgeführt wird.

Gegenstand der Erfindung ist daher ein Verfahren zur Herstellung von Pyromellitsäuredianhydrid in einem geschlossenen Gaskreislauf durch katalytische Gasphasenoxidation von 1,2,4,5-Tetraalkylbenzolen mit einem Sauerstoff enthaltenden Gas, dadurch gekennzeichnet, daß

a) die Desublimation des Pyromellitsäuredianhydrids mit Hilfe eines kalten, Sauerstoff enthaltenden Gases durchgeführt wird,

b) die in dem Abgas der Desublimation enthaltenden Nebenprodukte katalytisch verbrannt werden und

c) das Abgas der katalytischen Verbrennung der Nebenprodukte anteilig dem Reaktor für die Pyromellitsäuredieanhydrid-Herstellung zugeführt wird.

Das erfindungsgemäße Verfahren hat den Vorteil, daß die Desublimation des PMDA direkt mit einem Kühlgas durchgeführt werden kann, ohne die für das Gesamtverfahren pro Mengeneinheit PMDA benötigte Gasmenge zu erhöhen. Dadurch wird das Gesamtverfahren vereinfacht, zuverlässiger und, obwohl es ein Kühlgas zur Desublimation verwendet, billiger. Da ein Teil des Abgases aus der katalytischen Verbrennung in die Desublimation zurückgeführt werden kann, kann der Gasbedarf für das Gesamtverfahren sogar noch weiter reduziert werden. Ein weiterer Vorteil des Verfahrens ist, daß das Abgas aus der katalytischen Verbrennung so sauber ist, daß es direkt an die Atmosphäre abgegeben werden kann und ein hoher Schornstein entfällt. Gleichzeitig wird die Aufarbeitung des Abgases aus der Desublimation apparativ und verfahrenstechnisch wesentlich vereinfacht, die Beseitigung der Nebenprodukte entfällt, und durch die Ausnutzung der Verbrennungswärme der Nebenprodukte wird zusätzlich Energie gewonnen, so daß das Verfahren gegenüber dem Stand der Technik eine Reihe von ökonomischen und ökologischen Vorteilen bringt.

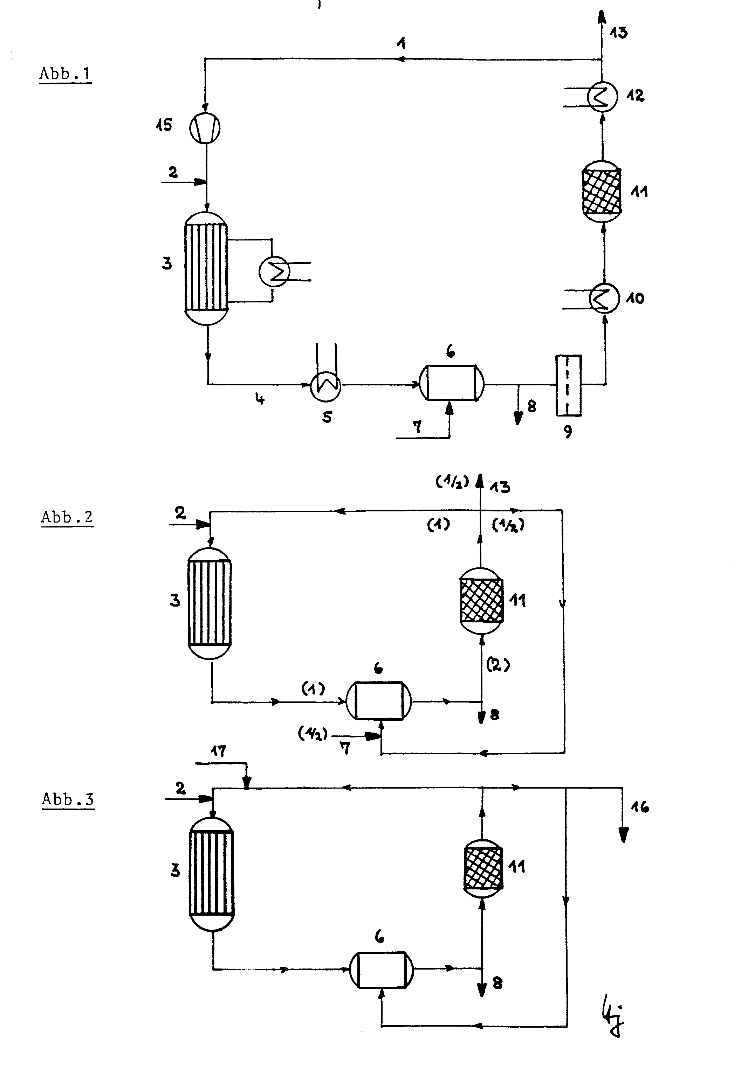

Das Verfahren wird anhand der Abbildungen 1 - 3 näher beschrieben.

Über die Leitung (1) (Abb. 1) wird heiße Luft, die aus dem Kreisprozeß stammt, in den Reaktor (3) gefahren. Vor dem Reaktor wird über die Leitung (2) Tetraalkyl-benzol in die heiße Luft gespritzt. Die Temperatur der Luft kann in einem sehr großen Bereich schwanken, sie sollte jedoch über dem Kondensationspunkt des Einsatzstoffes in der Luft liegen. Das Tetraalkyl-benzol kann in flüssiger Form in den Luftstrom eingespritzt werden, es kann aber auch in den gasförmigen Zustand überführt und dann mit der Luft gemischt werden. Das Verhältnis von Einsatzstoff zu Luft richtet sich nach dem verwendeten Katalysator und den gewünschten Temperatur- und Strömungsbedingungen im Katalysatorbett. In der Regel wird das Gewichtsverhältnis ca. 0,05 - 0,25 : 1 betragen.

Die Substituenten in den 1,2,4,5-Positionen des Benzols sind nicht auf Alkylgruppen beschränkt, sondern es kommen auch andere Substituenten in Frage, sofern sie ein Kohlenstoffatom aufweisen, das unmittelbar am Benzolring sitzt, und unter den Reaktionsbedingungen Carbonsäureanhydride bilden. Wegen der Vielzahl der möglichen Verbindungen muß im Einzelfalle geprüft werden, ob die Verbindungen für das Verfahren geeignet sind. Die Luft kann bei diesem Verfahren auch durch Sauerstoff ersetzt werden, der mit anderen, die Oxidationsreaktion, die Desublimation und die katalytische Abgasverbrennung nicht störenden inerten Gasen, wie z. B. Kohlendioxid und Wasserdampf, verdünnt wird. Bei der Verwendung anderer Verdünnungsgase für den Sauerstoff anstelle von Stickstoff muß im Einzelfalle geprüft werden, ob das Gas bei den nachfolgenden Schritten des Kreisprozesses nicht stört. Besonders bevorzugt wird als Kreisgas Luft.

Nach der Oxidation des Tetraalkyl-benzols im Reaktor (3) wird das Reaktionsgas, das PMDA und eine Reihe von Nebenprodukten enthält, über Leitung (4) in dem Wärmetauscher (5) auf eine Temperatur kurz oberhalb des Kondensationspunktes des PMDA im Reaktionsgas abgekühlt. Der Kondensationspunkt selbst hängt von der Konzentration des PMDA im Reaktionsgas ab, die ihrerseits von der Beladung der Luft mit Tetraalkyl-benzol vor dem Reaktor (3) und den Reaktionsbedingungen in dem Reaktor (3) abhängt.

Das vorgekühlte Reaktionsgas wird in dem Desublimator (6) durch Mischen mit einem kühlen Gas (7) auf eine Temperatur abgekühlt, die unterhalb des Kondensationspunktes des PMDA und oberhalb der Kondensationspunkte der Nebenprodukte liegt. Die einzustellende Desublimationstemperatur hängt von der Konzentration des PMDA im Reaktionsgas, dem Kühlgas, dem gewünschten Abscheidegrad für PMDA und der Art und Menge an Nebenprodukten im Reaktionsgas ab. Für die Abkühlung des Reaktionsgases kommen Gase in Frage, die unter den Desublimationsbedingungen nicht mit dem PMDA reagieren und bei der nachfolgenden katalytischen Nachverbrennung und bei der Oxidation des PMDA-Rohstoffes nicht stören. Bevorzugt werden sauerstoffhaltige Gase, da auf diese Weise der für die PMDA-Herstellung benötigte Sauerstoff in den Kreisprozeß eingeführt werden kann, z. B. Sauerstoff und Stickstoff und/oder Kohlendioxid und/oder Wasserdampf. Besonders bevorzugt wird als Kühlgas Luft. Vorteilhaft kann auch sein, sauerstoffhaltige Abgase aus anderen Produktionsanlagen zu verwenden, wenn sie Stoffe enthalten, die in der katalytischen Nachverbrennung verbrannt werden können und bei der Desublimation nicht stören, wie z. B. leichtsiedende Kohlenwasserstoffe und Kohlenmonoxid. Gleichzeitig wird durch den Einsatz dieser Abgase das Abgasproblem der entsprechenden Produktionsanlage entschärft. Die benötigte Menge an Kühlgas richtet sich nach der gewünschten Desublimationstemperatur und der Temperatur des Kühlgases. Sie sollte möglichst klein gehalten werden, um nicht zu große Gasmengen bewegen zu müssen. Die Temperatur des Kühlgases muß selbstverständlich unter der gewählten Desublimationstemperatur liegen. Da umso kleinere Kühlgasmengen benötigt werden, je tiefer die Temperatur des Kühlgases ist, werden tiefere Temperaturen bevorzugt. Besonders bevorzugt werden Gase mit Raumtemperatur. Es sind aber auch Gase mit tieferen Temperaturen als Raumtemperatur einsetzbar. Da diese Gase jedoch vor ihrem Einsatz erst abgekühlt werden müssen und Kühlenergie teuer ist, ist eine Abkühlung der Gase auf eine Temperatur unter Raumtemperatur in der Regel nicht sinnvoll. Bei der Verwendung von Kühlgasen mit Raumtemperatur liegt das Verhältnis Kühlgas zu Reaktionsgas in Abhängigkeit vom PMDA-Gehalt im Reaktionsgas, dem gewünschten Abscheidegrad für PMDA und der geforderten Reinheit des PMDA in der Regel bei 0,4 - 1,5 : 1 und besonders bevorzugt bei ca. 1 : 1.

Das in dem Desublimator (6) desublimierte PMDA wird mit Hilfe des Filters (9) von dem Gas getrennt und über die Leitung (8) ausgeschleust. Das Gas, das noch sämtliche Nebenprodukte enthält, wird in dem Wärmetauscher (10) auf eine Temperatur gebracht, die ausreicht, die katalytische und adiabatische Verbrennung der Nebenprodukte im Reaktor (11) in Gang zu setzen. Das Abgas aus der katalytischen Nachverbrennung enthält neben den Gasbestandteilen des Kühlgases für die Desublimation noch Kohlendioxid und Wasserdampf aus der Hauptreaktion im Reaktor (3) und der Nachverbrennung der Nebenprodukte im Reaktor (11).

Das Abgas aus dem Reaktor (11) ist in der Regel so sauber, daß nach der Rückgewinnung der Verbrennungswärme der Nebenprodukte in dem Wärmetauscher (12) ein Teil über die Leitung (13) in die Atmosphäre abgegeben wird und der andere Teil über die Leitung (1) und dem Kompressor (15) dem Reaktor (3) wieder zugeführt wird, so daß ein geschlossener Gaskreislauf entsteht. Die Aufteilung der beiden Gasströme (13) und (1) richtet sich nach dem Verhältnis von Reaktionsgas zu Kühlgas im Desublimator (6). Für das besonders bevorzugte Verhältnis von ca. 1 : 1 ist auch das Splittingverhältnis der beiden Gasströme (13) und (1) ca. 1 : 1. Anstatt das gesamte Abgas aus der katalytischen Nachverbrennung abzukühlen, ist es auch möglich, nur das auszuschleusende Abgas abzukühlen und das Kreisgas mit der Temperatur über die Leitung (1) in den Reaktor (3) zu fahren, mit der es aus dem Nachverbrennungsreaktor (11) kommt.

Mit Hilfe dieses erfindungsgemäßen Gaskreislaufes ist es möglich, die Desublimation des PMDA mit einem Kühlgas durchzuführen, ohne den Gesamtbedarf des Prozesses an Gasen zu erhöhen. Während bei dem bisher angewandten Verfahren des Standes der Technik die Luft vor dem Reaktor zugegeben und ausschließlich für die Oxidation des Alkylaromaten benötigt wird, hat bei dem erfindungsgemäßen Verfahren das sauerstoffhaltige Gas, das erst in der Desublimationsstufe in den Prozeß eingeführt wird, eine Doppelfunktion. Es dient zum einen als Kühlgas für die Desublimation und zum anderen ist es der Sauerstoff-Lieferant für die Hauptreaktion (PMDA-Herstellung) und die katalytische Abgasverbrennung (Verbrennung der Nebenprodukte). Bei dem Verfahren der Technik wird das Reaktionsgas nach der Desublimation des PMDA an gekühlten Flächen einer aufwendigen und in ihrer Wirkung nicht zufriedenstellenden Abgaswäsche unterworfen und anschließend ins Freie gegeben. Das erfindungsgemäße Verfahren unterwirft das Abgas aus der an sich bekannten Desublimation mit einem Kühlgas einer an sich bekannten katalytischen Nachverbrennung. Hierdurch wird ein sauberes Abgas erzeugt und gleichzeitig noch die Verbrennungswärme der Nebenprodukte gewonnen. Das Gas aus der katalytischen Nachverbrennung wird erfindungsgemäß geteilt, ein Teil wird zum Reaktor für die PMDA-Erzeugung zurückgeführt, während der andere Teil in die Atmosphäre abgegeben wird. Mit dem ins Freie geführten Abgasstrom werden auch die in den beiden Reaktoren des Verfahrens gebildeten Mengen an Kohlendioxid und Wasser ausgeschleust. Durch die Rückführung eines Teiles des Abgases in den Hauptreaktor ist es bei dem erfindungsgemäßen Verfahren möglich, dem für die Desublimation eingesetzten Kühlgas die gewünschte Doppelfunktion zu geben, Kühlgas und Sauerstoff-Lieferant zu sein, so daß trotz der Desublimation des PMDA mit einem Kühlgas gegenüber dem bisher verwendeten Verfahren keine zusätzlichen Gasmengen erforderlich sind.

Neben dem beschriebenen und bevorzugten Kreisprozeß gibt es noch einige im Rahmen der Erfindung liegende Varianten des Verfahrens. So kann z. B. bei dem in Abb. 2 stark vereinfacht wiedergegebenen Prozeß ein Teil des Abgases aus der katalytischen Nachverbrennung nach Kühlung zur Desublimation zurückgeführt werden, so daß zwei geschlossene, im Bereich der Desublimation und Nachverbrennung gemeinsam verlaufende Kreisläufe entstehen. Eine mögliche Aufteilung der Gasströme ist in Abb. 2 mitaufgeführt. Das rückgeführte Kreisgas für die Desublimation wird sinnvollerweise mit der kalten Luft gemischt. Die Luft kann jedoch prinzipiell auch an einer anderen Stelle der beiden Kreisläufe, z. B.

vor dem Reaktor zugegeben werden. In diesem Fall muß der rückgeführte Abgasstrom entsprechend größer sein. Die Rückführung eines Teiles des Abgases in die Desublimationsstufe hat den Vorteil, daß insgesamt weniger Luft benötigt wird, aber den Nachteil, daß der Sauerstoff-Partialdruck im Kreisgas für die PMDA-Erzeugung etwas niedriger als bei dem bevorzugten Verfahren ist.

Bei einer weiteren Variante des erfindungsgemäßen Verfahrens (Abb. 3) wird Stickstoff und/oder Kohlendioxid als Kreisgas für beide Kreisläufe, den Gaskreislauf für die Hauptreaktion und den Gaskreislauf für die Desublimation verwendet und das während der Hauptreaktion und der Nachverbrennung der Nebenprodukte gebildete Kohlendioxid und Wasser (Abb. 3, 16) aus dem Gaskreislauf für die Desublimation mit Hilfe in der Technik bekannter Methoden ausgeschleust. Der für die beiden Reaktionen benötigte Sauerstoff (Abb. 3, 17) wird in reiner Form zweckmäßigerweise vor dem Reaktor zur Erzeugung des PMDA zugeführt.

Patentansprüche:

1. Verfahren zur Herstellung von Pyromellitsäuredianhydrid in einem geschlossenen Gaskreislauf durch katalytische Gasphasenoxidation von 1,2,4,5-Tetraalkylbenzolen mit einem Sauerstoff enthaltenden Gas,
dadurch gekennzeichnet,
daß

    a) die Desublimation des Pyromellitsäuredianhydrids mit Hilfe eines kalten, Sauerstoff enthaltenden Gases durchgeführt wird,

    b) die in dem Abgas der Desublimation enthaltenen Nebenprodukte katalytisch verbrannt werden und

    c) das Abgas der katalytischen Verbrennung der Nebenprodukte anteilig dem Reaktor für die Pyromellitsäuredianhydrid-Herstellung zugeführt wird.

2. Verfahren nach Anspruch 1,
dadurch gekennzeichnet,
daß das Kreisgas und das Desublimationsgas gleiche oder verschiedene Zusammensetzungen aufweisen.

3. Verfahren nach Anspruch 1,
dadurch gekennzeichnet,
daß als Kreisgas für die Reaktion und als Desublimationsgas Sauerstoff und Stickstoff und/oder Kohlendioxid und/oder Wasserdampf verwendet wird.

4. Verfahren nach Anspruch 3,
dadurch gekennzeichnet,
daß als sauerstoffhaltiges Gas Luft verwendet wird.

0163851
O.Z. 3990

5. Verfahren nach Anspruch 1,
   dadurch gekennzeichnet,
   daß für die Desublimation des Pyromellitsäuredianhydrids
   ein sauerstoffhaltiges Gas verwendet wird, das organische Verbindungen enthält, die die Desublimation nicht
   stören, die katalytische Verbrennung der Nebenprodukte
   aber positiv beeinflussen.

6. Verfahren nach Anspruch 1,
   dadurch gekennzeichnet,
   daß ein Teil des Abgases aus der katalytischen Verbrennung der Nebenprodukte für die Desublimation des
   Pyromellitsäuredianhydrids verwendet wird, so daß ein
   zweiter, geschlossener Gaskreislauf entsteht.

7. Verfahren nach Anspruch 6,
   dadurch gekennzeichnet,
   daß das Kreisgas für die Reaktion und das Kreisgas
   für die Desublimation verschiedene Zusammensetzungen
   aufweisen.

0163851

o.z. 3990

Abb.1

Abb.2

Abb.3